# EUROPEAN PATENT SPECIFICATION

(11) **EP 1 408 837 B1**
(45) Date of publication and mention of the grant of the patent: **09.01.2008**
(21) Application number: 02716866.5
(22) Date of filing: 23.04.2002
(51) Int. Cl.: A61B 8/00

(54) **MEASURING DEVICE FOR EXAMINING A COMPRESSIBLE TISSUE**
MESSVORRICHTUNG ZUR UNTERSUCHUNG EINES KOMPRIMIERBAREN GEWEBES
DISPOSITIF DE MESURE PERMETTANT D'EXAMINER DES TISSUS COMPRESSIBLES

(30) Priority: 23.04.2001 FI 20010832
(43) Date of publication of application: 21.04.2004
(73) Proprietor: Jurvelin, Jukka, 70150 Kuopio (FI); KIVIRANTA, Ilkka, SF-70620 Kuopio (FI); Toyras, Juha, 70820 Kuopio (FI)
(72) Inventor: Jurvelin, Jukka, 70150 Kuopio (FI); KIVIRANTA, Ilkka, SF-70620 Kuopio (FI); Toyras, Juha, 70820 Kuopio (FI)
(74) Representative: Helino, Timo Kalervo
(86) International application number: PCT/FI2002/000334
(87) International publication number: WO 2002/085214

(56) References cited:
- EP-A1- 0 920 833
- WO-A1-93/02619
- JUN-KYO SUH ET AL: 'Determination of the mechanical properties of articular cartilage using a high-frequency ultrasonic indentation technique' 1999 BIOENGINEERING CONFERENCE, BIG KSY, [Online] 16 June 1999 - 20 June 1999, MONTANA, pages 1 - 2, XP002978866 Retrieved from the Internet: <URL:http://asme.pinetec.com/biol1999> [retrieved on 2002-07-23]
- JUN-KYO FRANCIS SUH ET AL: 'An in situ calibration of an ultrasound transducer: a potential application for an ulstrasonic indentation test of articular cartilage' JOURNAL OF BIOMECHANICS vol. 34, 2001, pages 1347 - 1353, XP002959023
- YONGPING ZHENG ET AL: 'Objective assessment of limb tissue elasticity: Development of a manual indentation procedure' JOURNAL OF REHABILITATION RESEARCH AND DEVELOPMENT vol. 36, no. 2, April 1999, pages 71 - 85, XP002978859

## Description

The present invention relates to a measuring device as defined in the preamble of claim 1 for examining a compressible tissue, such as articular cartilage.

Articular cartilage consists of differentiated connective tissue that contains no blood vessels, lymphatic vessels or nerves. Articular cartilage is the stiffest kind of soft tissue in the human body, yet it is clearly softer than bone. The thickness of articular cartilage varies in different joints from a few micrometers to several millimeters. The thickness of articular cartilage may also be different in different parts of joint surfaces. Articular cartilage has two main functions - reducing the stress applied to the subchondral bone and reducing friction in the joint.

The properties of articular cartilage, including mechanical properties, undergo changes during articular cartilage diseases such as arthrosis, rheumatoid arthritis and chondromalacia. Softening of cartilage is often the first perceptible symptom of cartilage decay. On the other hand, e.g. after a corrective treatment of an articular cartilage injury by cellular transfer, the corrective tissue will gradually solidify, allowing measurement of mechanical properties of the tissue to be used for the estimation of the results of surgery.

The simplest method used for estimating the stiffness of articular cartilage is to feel the cartilage surface by pressing on it with a metallic instrument in connection with an operation. However, the results of such measurement are highly subjective and inconsistent.

Various measuring devices have been developed for use in connection with surgical operations. Prior-art technology in the field of the invention is represented by patent FI 90616. A tissue stiffness measuring device disclosed in this specification comprises an elongated rigid frame comprising a contact surface which, in connection with a measurement, is pressed against the surface of the tissue being examined. The frame supports a measuring arm provided with a projecting measuring stud or an equivalent, relatively small pin. The measuring arm is also provided with a sensor for the measurement of the stress applied to it via the measuring stud from the tissue being measured.

When this measuring device is used, its contact surface is pressed against the tissue to be measured so that the measuring stud projecting outside the contact surface compresses the cartilage under it. Thus, the cartilage exerts a pressure proportional to its stiffness on the measuring stud and the measuring arm. This pressure produces measurable stresses in the measuring arm. Corresponding measurable stresses are produced in the frame when the contact surface is pressed against the cartilage to be measured.

This prior-art measuring device has proved to be a very workable and effective tool in the examination of compressible tissues, such as articular cartilage. The main problem with this prior-art device is only the scantness of information produced by it. The device was so designed that the effect of cartilage thickness could be minimized, because the device could not be used for the measurement of cartilage thickness. In investigations concerning cartilage tissue, there is clearly a need to obtain other information about the tissue in addition to its stiffness, which does not tell all about the tissue being examined. Thus, a need has arisen to develop a measuring device that can be used to accomplish, in addition to arthroscopic mechanical stiffness measurement of the tissue, a more detailed characterization of the tissue structure.

The closest prior art EP-A-0920833 discloses a measuring device for examining a compressible tissue with an elongated rigid frame, a measuring arm, an ultrasound probe as a measuring stud and means for processing signals obtained from the stud.

As for the features characteristic of the invention, reference is made to the claims.

By examining e.g. articular cartilage in this manner, it is possible to determine the thickness of the cartilage layer, the ability of the cartilage surface, internal structures and subchondral bone to reflect or scatter the sound as well as the attenuation of ultrasound as a function of frequency.

Of the above-mentioned acoustic parameters:
- Cartilage layer thickness is understandably an important parameter in an estimation of the condition of cartilage.
- The ability of cartilage surface and internal structures of cartilage to reflect/scatter ultrasound are a sensitive indication of cartilage quality and especially of the condition of collagenous fibers on cartilage surface.
- There is no wide experience about the application of frequency-dependent attenuation to the estimation of cartilage condition, but this parameter (BUA) has proved to be useful in the estimation of bone quality. It may be useful in the estimation of cartilage quality as well.
- Back-scattering parameters have been successfully applied in the estimation of both cartilage and bone quality.
- The ability of subchondral bone to reflect/scatter ultrasound is a sensitive indication of bone quality.

The device of the invention for the measurement of compressible tissue, such as articular cartilage, comprises an elongated rigid frame and a measuring arm supported by the frame. Th arm can additionally be provided with a contact surface to be placed against the tissue to be examined. The device further comprises a measuring stud and means for processing signals obtained from the measuring stud. According to the invention, the measuring stud is an ultrasound crystal matrix, i.e. a set of ultrasonic probes that together form an area covering all or part of the tissue to be examined for emitting ultrasound into the tissue to be examined and for receiving ultrasound from the tissue to be examined.

Thus, instead of a scan, the probe matrix can provide information about a desired area of the tissue.

The measuring device of the invention can be successfully used for quantitative determination of mechanical and structural properties of articular cartilage in connection with arthroscopy. Extending acoustic measurements even to the bone under the cartilage allows quantitative determination of the properties of the subchondral bone. It is also very important to know the properties of the subchondral bone because changes in said properties have been found to bear a relation to degeneration of cartilage.

The measuring device of the invention is particularly advantageous in measurements of the echo reflected from a cartilage-bone interface, which provides cartilage thickness and compression information as well as, on the basis of force measurements obtained, material parameters descriptive of the tissue.

After the measurements according to the invention, an actual analysis and diagnosis can be made by combining all measured parameters and the information obtained from them. A diagnosis is preferably made by collecting extensive reference material about all corresponding measurements to be performed and entering the new measured data into such a file containing reference material, whereupon it is possible to obtain an estimate of the condition of the joint on the basis of the previously collected reference material.

The measuring device of the invention has significant advantages as compared with prior art. The invention makes it possible to achieve measurement of actual material properties of cartilage, measurement of structural and compositional properties of cartilage and subchondral bone and measurement of cartilage thickness. Moreover, variations in cartilage thickness have no effect on the measurement results as in prior-art methods.

In the following, the invention will be described in detail with reference to the attached drawings, wherein
Fig. 1 presents a general view of a measuring device according to the invention,
Fig. 2 illustrates an ultrasound crystal structure used in the invention, and
Fig. 3 presents a general view of a second measuring device according to the invention.

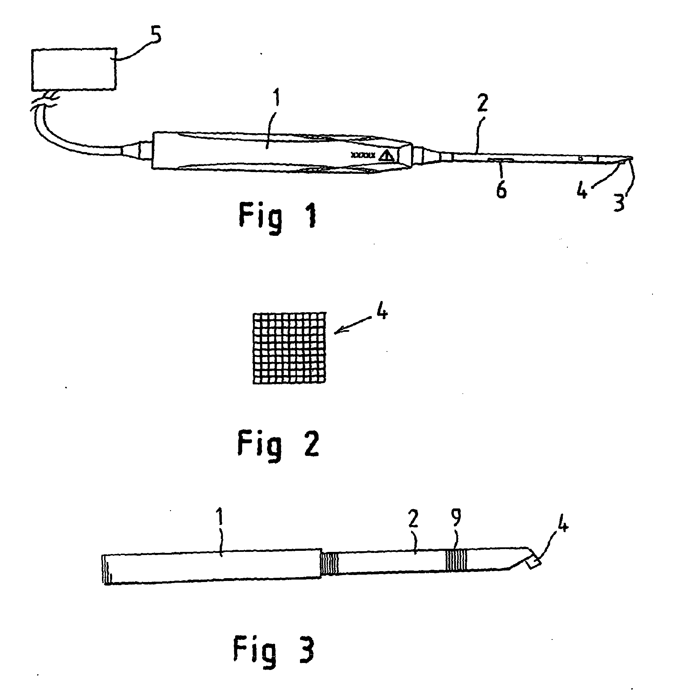

The measuring device presented in Fig. 1 comprises an elongated, rigid frame 1, i.e. a handle, and a straight and rigid measuring arm 2 attached to the frame. The beveled outer end of the measuring arm comprises a contact surface 3 to be placed against the tissue to be examined, with a measuring stud 4 protruding from this surface. In addition, the measuring arm is provided with at least one sensor, preferably a strain gauge, for measuring the force applied via the measuring stud from the tissue being examined to the measuring arm. The measuring device is connected to suitable means 5 for processing the signals detected by the measuring arm.

As presented in Fig 2, the ultrasound probe 4 is an ultrasound crystal matrix consisting of a plurality of separate ultrasound crystals. The matrix covers the tissue area to be examined at a time and the matrix is not moved during the examination. The measurement is performed one matrix element or crystal at a time so that measurements by different crystals do not interfere with each other. Thus, point-specific measurements uniformly covering the entire matrix area of the tissue to be examined are obtained.

When the ultrasound probe is held in a projecting position as in Fig. 1, point-specific information about the tissue can be obtained by pressing the probe 4 into the tissue. Thus, a device according to the invention may be an embodiment as illustrated in Fig. 1, wherein the ultrasound probe 4 is fixedly mounted and continuously protruding out of the oblique contact surface 3. In this case, the tissue to be examined will return a point-specific acoustic signal that provides significant information especially about the bone and the bone interface in the form of possible fading or enhancement of reflection or scattering.

In the embodiment presented in Fig. 3, the measuring device is a pen-like structure in which a rigid frame 1 and a thinner measuring arm 2 extending from it are connected together. The end of the measuring arm is provided with an ultrasound probe 4 mounted in an oblique position relative to the longitudinal direction of the arm. There is no contact surface at the end of the measuring arm, but the device is used by only pressing the probe against the tissue to be examined. Placed in the measuring arm relatively close to the probe 4 are strain gauges or equivalent sensors 9 for measuring the force applied to press the ultrasound probe against the tissue. Thus, the device produces point-like measurement results for the tissue to be examined.

Thus, when the device of the invention is used to examine tissue such as cartilage and subchondral bone, both mechanical, structural and compositional properties of the tissue can be determined by the same device in connection with arthroscopy.

## Claims

1. Measuring device for examining a compressible tissue, such as articular cartilage, said device comprising an elongated rigid frame (1), a measuring arm (2) attached to the frame and a measuring stud (4), said measuring stud being an ultrasound probe, and means (5) for processing signals obtained from the measuring stud, **characterized in that** the measuring stud (4) is an ultrasound crystal matrix that substantially covers the tissue area to be examined for emitting ultrasound into the tissue to be examined and receiving ultrasound from the tissue to be examined.

2. Measuring device according to claim 1, **characterized in that** the measuring device comprises a contact surface (3) that can be placed against the tissue to be examined.

## Patentansprüche

1. Messvorrichtung zur Untersuchung eines komprimierbaren Gewebes, wie Gelenkknorpel, wobei die Vorrichtung einen länglichen starren Körper (1), einen an dem Körper angebrachten Messarm (2) und einen Messkopf (4), wobei der Messkopf eine Ultraschallsonde ist, und Mittel (5) zur Verarbeitung von von dem Messkopf erhaltenen Signalen umfasst, wobei die Messvorrichtung **dadurch gekennzeichnet ist, dass** der Messkopf (4) eine Ultraschallkristallmatrix, die die zu untersuchende Gewebefläche im wesentlichen bedeckt, zur Emission von Ultraschall in das zu untersuchende Gewebe und zum Empfangen von Ultraschall von dem zu untersuchenden Gewebe ist.

2. Messvorrichtung nach Anspruch 1, die **dadurch gekennzeichnet ist, dass** die Messvorrichtung eine Kontaktoberfläche (3), die gegen das zu untersuchende Gewebe gelegt werden kann, umfasst.

## Revendications

1. Appareil de mesure pour l'examen d'un tissu compressible, tel un cartilage articulaire, ledit dispositif comprenant un châssis rigide allongé (1), un bras de mesure (2) fixé au châssis et un embout de mesure (4), ledit embout de mesure étant une sonde à ultrasons, et des moyens (5) de traitement des signaux obtenus depuis l'embout de mesure, **caractérisé en ce que** l'embout de mesure (4) est une matrice de cristaux à ultrasons qui recouvre sensiblement la zone de tissu à examiner, destinée à émettre des ultrasons dans le tissu à examiner et à recevoir des ultrasons du tissu à examiner.

2. Appareil de mesure selon la revendication 1, **caractérisé en ce que** l'appareil de mesure comprend une surface de contact (3) qui peut être placée contre le tissu à examiner.
